(19) Europäisches Patentamt
European Patent Office
Office européen des brevets

(11) Publication number: 0 311 366 B1

(12) EUROPEAN PATENT SPECIFICATION

(45) Date of publication of patent specification: 06.04.94

(51) Int. Cl.[5]: C07D 471/04, A61K 31/435, //(C07D471/04,221:00,205:00)

(21) Application number: 88309261.1

(22) Date of filing: 05.10.88

The file contains technical information submitted after the application was filed and not included in this specification

# (54) Crystalline beta-lactam hydrate.

(30) Priority: 06.10.87 US 105766

(43) Date of publication of application: 12.04.89 Bulletin 89/15

(45) Publication of the grant of the patent: 06.04.94 Bulletin 94/14

(84) Designated Contracting States: AT BE CH DE ES FR GB GR IT LI LU NL SE

(56) References cited:
US-A- 3 655 656
US-A- 4 335 211

(73) Proprietor: ELI LILLY AND COMPANY
Lilly Corporate Center
Indianapolis Indiana 46285(US)

(72) Inventor: Pasini, Carol Elaine
1017 New Amsterdam Drive
Greenwood Indiana 46142(US)

(74) Representative: Hudson, Christopher Mark et al
Erl Wood Manor
Windlesham Surrey GU20 6PH (GB)

EP 0 311 366 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention relates to a novel monohydrate of a β-lactam antibiotic, more particularly to a novel crystalline monohydrate form of a 1-carbacephalosporin.

The β-lactam antibiotic of the formula I

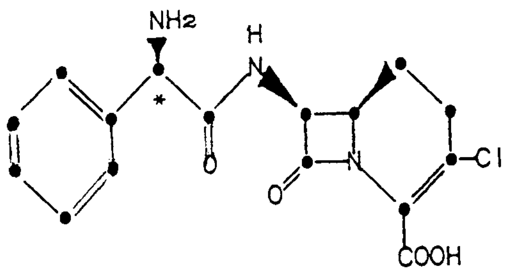

I

where the asterisk indicates R-absolute stereochemistry, is a potent new orally-active antibiotic. The antibiotic is described, for example, in U.S. Patent No. 4,335,211. For brevity's sake, the anhydrous form of the compound of the above formula will be referred to by the Lilly serial number LY163892.

The present invention is directed to the crystalline monohydrate of LY163892. LY163892 crystalline monohydrate is a pharmaceutically elegant hydrate of the parent compound. The crystalline monohydrate is stable over a wide relative humidity range, (i.e., approximately 10% to approximately 70%) and especially in the humidity range encountered in production and subsequent storage (approximately 30%) of the finished (commercial) form containing the compound. In contrast, the di-, tri- and tetra-hydrate forms will either lose or gain water at this humidity range, making these forms undesirable for use in pharmaceutical formulations. The crystalline monohydrate also has a superior bulk density to the other hydrates (such as the trihydrate and tetrahydrate), and thus is a superior form for use in capsules, an important advantage over the other hydrate forms.

The LY163892 crystalline monohydrate of the invention has the following X-ray powder diffraction pattern:

Table

| d | $I/I_1$ |
|---|---|
| 18.79 | .75 |
| 12.89 | .40 |
| 9.30 | .80 |
| 8.42 | .05 |
| 7.75 | .50 |
| 7.37 | .15 |
| 6.46 | .32 |
| 6.10 | .03 |
| 5.86 | .03 |
| 5.66 | .10 |
| 5.15 | .25 |
| 4.84 | .45 |
| 4.70 | .40 |
| 4.29 | 1.00 |
| 4.06 | .35 |
| 3.89 | .20 |
| 3.77 | .28 |
| 3.69 | .37 |
| 3.53 | .13 |
| 3.49 | .13 |
| 3.39 | .11 |
| 3.24 | .10 |
| 3.13 | .05 |
| 3.05 | .16 |
| 2.95 | .03 |
| 2.90 | .04 |
| 2.85 | .06 |
| 2.77 | .03 |

The above pattern was obtained with nickel-filtered copper radiation (Cu:Ni) of wavelength $\lambda$ = 1.5418 Å. The interplanar spacings are in the column denoted as "d" and the relative intensities are in the column "$I/I_1$".

The compound of Formula I can exist in the form of a zwitterion, and it is to be clearly understood that such a zwitterion is embraced by the invention.

The crystalline monohydrate is a white microcrystalline solid.

As discussed above and further evinced by Figure 1 in the accompanying drawing, the monohydrate has superior stability when compared to the di-, tri-, and tetrahydrate forms. The data for Figure 1 was collected by placing freshly prepared and tared samples (the moisture content of each form was obtained by a standard Karl Fisher analysis) into controlled-humidity jars at room temperature for a sufficient period of time for equilibration to occur (2 to 4 days). At the appropriate time, the samples were removed from the jars and re-tared. All weight loss or gain in the re-tared samples were attributed to the loss or gain of water. The percent of water in the sample versus the relative humidity of the jar in which the sample was stored was plotted for each hydrate form to generate Figure 1.

This invention also provides a process for preparing the crystalline monohydrate of the compound of Formula (I) which comprises adjusting the pH of an aqueous solution containing a compound of Formula (I) to about 2 to 6.

The monohydrate can be made from the anhydrate, various ethanol solvates, the various DMF solvates (or the various combination DMF solvate-hydrate), or from the dihydrate of LY163892. (The synthesis of several of such starting materials is discussed below in the Experimental Section.) The preferred starting material is either the anhydrate or the ethanol solvate of LY163892.

The above starting materials are first suspended in water. The most common procedure is to effect solution of the starting material by the addition of a minimum amount of acid, generally 6N (or more dilute) hydrochloric acid. Alternatively, a solution of the starting material is effected by adding the minimum amount of base (for example, the minimum amount of 2N sodium hydroxide, resulting in a pH of about 7.6).

Regardless of how solution is effected, crystallization is induced by slowly adjusting the pH of the solution of starting material to the desired range, preferably to approximately 4 to 5, and most preferably about 4.8. If the solution is effected by the addition of acid, it is preferred to raise the temperature of the solution to about 50°C and slowly add triethylamine (alternatively, sodium hydroxide) to the solution until a pH of approximately 4.8 is obtained. The gradually developing suspension is stirred and maintained at about 50°C during the addition of the base. Seeding the solution with a small amount of crystalline monohydrate early in the addition period of base is preferred. For example, seeding is often done when the pH of the solution is approximately 1.8. If the starting material solution was effected by the addition of base, the pH is slowly taken to approximately 4 by the addition of an acid (preferably 2N hydrochloric acid). Effecting solution of the starting material with hydrochloric acid and inducing crystallization by adjusting the pH of the solution to approximately 4.8 with triethylamine is preferred.

The suspension resulting from adjusting the pH of starting material solution to approximately 2 to 6 is isolated by conventional filtering techniques, such as vacuum filtration on a Büchner funnel. The collected crystals are washed and allowed to dry in air at ambient temperature. Alternatively, the warm pH-adjusted suspensions (50°C) are cooled to approximately 20°C, stirred, filtered (such as on Büchner funnel) and the collected solid dried 30°C for 24 to 48 hours by conventional means (such as a cleaned-air oven).

In a further aspect of this invention there is provided a pharmaceutical formulation comprising as an active ingredient a crystalline monohydrate of the compound of Formula (I) associated with one or more pharmaceutically acceptable carriers, vehicles or excipients therefor. These pharmaceutical compositions are useful for the control of gram-positive and gram-negative bacterial infections in warm-blooded animals.

With regard to compositions for oral administration (such as tablets and capsules), the term "suitable carrier, vehicle or excipient" includes common excipients such as binding agents, for example, syrup, acacia, gelatin, sorbitol, tragacanth, polyvinylpyrrolidine (Povidone), methylcellulose, ethylcellulose, sodium carboxymethylcellulose, hydroxypropylmethylcellulose, sucrose, and starch; fillers and carriers, for example corn starch, gelatin, lactose, sucrose, microcrystalline cellulose, kaolin, mannitol, dicalcium phosphate, sodium chloride, and alginic acid; disintegrators such as croscarmellose sodium, microcrystalline cellulose, corn starch, sodium starch glycolate, alginic acid and mutable wetting agents such as sodium lauryl sulfate; and lubricants such as magnesium stearate and other metallic stearates, stearic acid, silicone fluid, talc, waxes, oils and colloidal silica. Flavoring agents such as peppermint, oil of wintergreen, cherry flavoring or the like can also be used. It may be desirable to add a coloring agent to make the dosage form more aesthetically pleasing in appearance or to help identify the product. The tablets may also be coated by methods well known in the art.

The pharmaceutical compositions of the present invention may also be in the form of oral liquid preparations, which may be either a) aqueous or oily suspensions, solutions, emulsions or syrups; or b) a dry powder to be reconstituted with water or another suitable vehicle before use. When used in conjunction with such oral liquid preparations, the term "suitable carrier, vehicle or excipient" means conventional additives such as suspending agents such as acacia, methyl cellulose, hydroxyethyl cellulose, carboxymethyl cellulose, cellulose with sodium carboxymethyl cellulose (Avicel®), xantham gum, or starch; sweeteners such as sucrose, syrup, glucose, saccharin, sorbital, or aspartame; wetting agents such as sodium lauryl sulfate, silicone oil, the various Pluromics® surfactants, or glycerin; preservatives such as methyl, propyl, or butyl p-hydroxy benzoates, or sorbic acid; dyes, flavors and salts such as sodium chloride, citric acid, oil of wintergreen or sodium citrate; and waters, oils, and esters such as almond oil, fractionated coconut oil, hydrogenated caster oil, lecithin, aluminum stearate, and the like.

Topical compositions can be formulated with "suitable carriers, vehicles or excipients" such as hydrophobic or hydrophilic bases. Such bases include ointments, creams or lotions.

Veterinary pharmaceutical compositions of the antibiotic compounds may be administered in the feed or the drinking water of farm animals. Alternatively, the compounds can be formulated as intramammary preparations with "suitable carriers, vehicles or excipients" such as long- or quick-release bases.

The crystalline monohydrate of LY163892 can also be formulated in unit dosage form in sterile vials, sterile plastic pouches containing a port with a septum, or sterile, hermetically sealed ampoules. The amount of the crystalline monohydrate compound per unit dosage may vary from about 100 milligrams to about 10 grams. A preferred amount is from 200 to 500 milligrams per unit dosage.

A "therapeutically effective amount" of the crystalline monohydrate compounds of Formula I is from approximately 2.5 mg to about 70 mg of compound per kilogram of body weight per dose. This amount generally totals from about 200 mg to about 7 grams per day for an adult human.

During a course of treatment with the instant crystalline monohydrate antibiotic, the antibiotic compound can be administered in a single daily dose or in multiple doses per day. The treatment regime may require administration over extended periods of time, for example, for several days or for from two to three weeks.

The amount administered per dose or the total amount administered will depend on such factors as the nature and severity of the infection, the age and general health of the patient, and the tolerance to the crystalline monohydrate antibiotic compounds of Formula I of both the patient and the microorganism or microorganisms involved in the infection.

In the following Preparations and Examples, the terms dimethylformamide, nuclear magnetic resonance spectra, infrared spectroscopy are abbreviated DMF, n.m.r., and i.r., respectively.

In conjunction with the n.m.r. spectra, the following abbreviations are used: "s" is singlet, "d" is doublet, "dd" is doublet of doublets, "t" is triplet, "q" is quartet, and "m" is multiplet.

The n.m.r. spectra were obtained on a General Electric QE-300 300 MHz instrument. The chemical shifts are expressed in $\delta$ values (parts per million downfield from tetramethylsilane).

Experimental Section

Example 1

7$\beta$-[2′-(R)-2′-phenyl-2′-aminoacetamido]-3-chloro-3-(1-carba-1-dethiacephem)-4-carboxylic acid monohydrate from the corresponding anhydrate (LY163892).

(Example 1 teaches how to form the title compound from the corresponding anhydrate. Thus, the various procedures under "Step 1" teach how to make the amorphous anhydrate compound, and the various procedures under "Step 2" teach the conversion of amorphous anhydrate to the crystalline monohydrate. Also noted in the procedures are the presence of other intermediates solvates and hydrates formed in the course of the procedures).

Step 1

LY163892 Anhydrate

Step 1, Procedure A

Under a nitrogen atmosphere, DMF (10.5L) was warmed to 45 °C and 7$\beta$-amino-3-chloro-3-(1-carba-1-dethiacephem)-4-carboxylic acid zwitterion (583.5 g) and N,N′-Bis(trimethylsilyl)urea (1259.3 g, 6.16 mol) were added. The resultant mixture was stirred for 1 hour at 45 °C then cooled to -50 °C. Pyridine (397 ml, 4.90 mol) then 2-(R)-2-phenyl-2-aminoacetyl chloride, hydrochloride salt (550.2 g, 2.46 mol) was added over a 2 to 3 minute period and the resultant solution was stirred for 2 hours at between -45 °C to -35 °C. The mixture was cooled to -45 °C and 5N hydrochloric acid (49.16 ml) then water (1.05L) was added over a 10 minute period raising the temperature of the mixture to -45 °C to -25 °C. The mixture was stirred and warmed to 20 °C over a 30 minute period, yielding a mixture with a pH of 3.5. The mixture was filtered over Hyflo®. (The filtrate at this point contained 7$\beta$-[2′-(R)-2′-phenyl-2′-aminoacetamido-3-chloro-3-(1-carba-1-dethiacephem)-4-carboxylic acid, mono(dimethylformamide) dihydrate). The filter was rinsed with a minimal amount of 9:1 DMF:water solution. The filtrate (with the combined wash) was warmed to 50 °C and the pH of the solution was adjusted to 4.6 by the addition of triethylamine (400 ml). The mixture was seeded with some LY163892 mono(DMF) dihydrate then the pH of the solution was brought to 4.8 by the addition of triethylamine. The solution was stirred for 30 minutes to begin crystallization. Triethylamine (200 ml) was added to take the pH of the solution to 6.1. The solution was stirred for an additional 30 minutes at 50 °C to stabilize the pH. The resultant mixture was filtered over polypropylene through two (cleaned) Büchner filters. Each filter was washed with a 9:1 a DMF:water mixture (50 °C, 1.5L) then with acetonitrile (2X, 1L). The filter cakes were pulled dry on the Büchner filters then slurried in 3A ethanol (6L) for 30 minutes. The slurry was filtered on a 32 cm Büchner filter (the filtration took place over three hours). The collected solid was washed with 3A ethanol (2X, 1L) then a rubber dam was placed on the filter and a vacuum was applied on the filter overnight. The resultant solid was dried for 2 days in an air dryer at 30 °C to give 745 g, 73.7% yield (from theoretical base grams) of 7$\beta$-[2′-(R)-2′-phenyl-2′-aminoacetamido]-3-chloro-3-(1-carba-1-dethiacephem)-4-carboxylic acid anhydrate (LY163892 anhydrate).

Step 1, Procedure B

DMF (1L) was heated to 45 °C and 7$\beta$-amino-3-chloro-3-(1-carba-1-dethiacephem)-4-carboxylic acid zwitterion (60.0 g, 0.267 mole) was suspended therein. N,N′-Bis(trimethylsilyl)urea (136.9 g, 0.670 mole) was washed into the suspension with additional DMF (140 ml). The suspension was warmed to 45 °C to

give a gold solution. The solution was stirred at that temperature for 1 hour then cooled to -50°C. Pyridine (43.15 ml, 0.533 mole) and 2-(R)-2-phenyl-2-aminoacetyl chloride, hydrochloride salt (59.80 g, 0.267 mole) was added to the solution over a 10 minute period while the temperature of the solution was maintained at -48°C. The solution was stirred from between -46° to -44°C for 85 minutes to give a homogeneous solution. The solution was stirred for another 30 minutes then quenched with 5N hydrochloric acid (5.33 ml, 0.026 mole) and water (110 ml). (During quenching, the solutions temperature did not go above -30°C.) The solution was warmed to 9°C and filtered. The filtered solution was warmed to 50°C and the pH of the solution was increased to 4.2 over 34 minutes using a subsurface triethylamine pump. The solution was seeded with LY163892 anhydrate and the pH of the solution was increased to 4.33 over 1.5 minutes by the addition of triethylamine. The solution was stirred for an additional 30 minutes to give a thick slurry. The pH of the slurry was raised to 6.2 over 24 minutes by the addition of triethylamine. 1N hydrochloric acid was added to the slurry to adjust the pH to 6.06 and the resultant slurry was stirred for 10 minutes then filtered. The filter cake was washed with a 9:1 DMF:water mixture (500 ml) then dried in the Büchner funnel overnight. The filter cake was slurried in 3A ethanol (1150 ml), filtered and dried at 45°C for 18 hours to yield 70.11 g of the LY163892 anhydrate (slightly contaminated with ethanol).

Step 2

LY163892 Monohydrate

Step 2, Procedure A

Water (filtered, purified, 8L) and 7$\beta$-[2′-(R)-2′-phenyl-2′-aminoacetamido]-3-chloro-3-(1-carba-1-dethiacephem)-4-carboxylic acid anhydrate (1447.5 g) were combined and stirred for 30 minutes at 15°C. To the resultant slurry was added hydrochloric acid (6N, 333 ml in 1L water). The slurry was stirred for 30 minutes then an additional amount of hydrochloric acid (20 ml) was added to effect complete solution. The solution was stirred for 20 minutes past the final addition of hydrochloric acid. Darco® G60 carbon black (750 ml) and (tetrasodium)(ethylenediaminetetraacetate) (32 g) was added and the suspension was stirred for 30 minutes. The suspension was filtered over a Hyflo® filter aid pad then the filter pad was washed with water (750 ml). The combined filtrate and washes were stirred and warmed to 50°C over 15 minutes. The pH of the filtrate was adjusted to 1.65 with triethylamine while maintaining the temperature of the filtrate between 48° to 49°C. The resultant solution was seeded with 100 mg of LY163892 monohydrate. The resultant pH of the seeded mixture was adjusted to 1.8 over the next 8 minutes by the addition of triethylamine. The mixture was stirred slowly for 1 hour to allow crystallizaton to occur. The pH of the slurry was adjusted to 4.80 by the addition of triethylamine while maintaining the temperature of the suspension between 50° and 52°C. The suspension was stirred for 25 minutes then cooled to 20°C over a 1 hour period. The suspension was filtered over polypropylene on a 32 cm Büchner filter. The filter was covered with a dam and vacuum was maintained on the filter cake overnight. The filter cake was cut from filter and stirred for 1 hour in water (4L) at 5°C. The suspension was filtered, the filter was washed with filtered, purified water at 5°C (2X, 1L) and the filter cake was covered and vacuum was applied to the filter cake for 2 hours. The filter cake was cut from the filter and put in a cleaned air oven to be dried for 48 hours at 30°C. This procedure yielded 958.1 g of the LY163892 monohydrate.

Step 2, Procedure B

7$\beta$-[2′-(R)-2′-Phenyl-2′-aminoacetamido]-3-chloro-3-(1-carba-1-dethiacephem)-4-carboxylic acid anhydrate (206.27 g) was suspended in water (15°C, 1.07L) with stirring. Hydrochloric acid (45 ml of 12N in 135 ml of water) was added and the suspension was stirred 30 minutes to effect solution (requiring the addition of 2 ml more of hydrochloric acid). The solution was filtered through glass fiber filter paper, and the fiber paper was washed with water (50 ml). The filtrate was warmed to 50°C. Triethylamine was added to the filtrate (maintained at approximately 50°C) over a period of 80 minutes to raise the pH of the solution to 4.8. The solution was stirred for an additional 30 minutes at 50°C then cooled to 20°C and stirred for 45 minutes. The resultant precipitate was collected by filtration on a 24 cm Büchner funnel. The filter cake was washed with water (100 ml) and the cake was cut from the filter. The filter cake was added to cold (5°C) water (600 ml). The resultant slurry was stirred at 5°C for 30 minutes. The slurry was then filtered, the filter cake was washed with cold (5°C) water (2X, 100 ml), and the filter cake was dried in vacuo on the funnel overnight. The filter cake was air dried in a cleaned air oven at 30°C for 24 hours to yield 131.44 g, 74% yield of the LY163892 monohydrate.

Step 2, Procedure C

Water (9L) and hydrochloric acid (410 ml of 12N in 1L water) were combined. LY163892 anhydrate was added and the suspension was stirred for 15 minutes. An additional amount of hydrochloric acid (10 ml) was added and the solution was stirred for 1 hour at 16°C to effect solution. The solution was filtered through glass fiber pad on a 24 cm cleaned table-top funnel, the funnel was rinsed with water (500 ml) then the filtrate transferred to a cleaned flask rinsing with double filtered water. The filtrate was heated to 50°C over 1.25 hours. To the warmed filtrate triethylamine was added over a 10 minute period to raise pH of the solution to 1.55. The solution was seeded with LY163892 monohydrate, and the solution's pH was brought to 1.85 over 40 minutes by the addition of triethylamine. The resultant slurry was stirred for 40 minutes at 50°C then brought to pH 4.8 by the addition of triethylamine over a 45 minute period. The resultant suspension was stirred at 50°C for 25 minutes then cooled to 22°C over 1 hour. The suspension was filtered on a cleaned 32 cm Büchner funnels and a cleaned 24 cm Büchner funnel. The filters were washed with water (room temperature, a total of one liter). The filter cake on both filters was dried in vacuo for 15 minutes. The filter cake was cut from both filters and combined with water (5.0L). The resultant suspension was stirred at 5°C for 30 minutes then filtered over polypropylene on a 32 cm Büchner funnel. The funnel was covered and vacuum was applied to the filter cake overnight. The filter cake was washed with cold (5°C) water (2X, 1L). The filter cake was dried in vacuo (1.5 hours) then cut from the filter and dried in a cleaned air oven at between 25 to 30°C for 2 days to yield 1088.8 g of LY163892 monohydrate. n.m.r. (300 MHz, $D_2O$-DCl) δ: 1.33 (m), 1.62 (m), 2.60 (m), 3.95 (q), 5.03 (s), 5.25 (s), 5.45 (d), 7.57 (s); i.r. (KBr) (β-lactam region) 1790, 1750, 1680, 1600.

Example 2

LY163892 monohydrate from the corresponding mono(dimethylformamide) dihydrate form.

Water (9.75L) was filtered into a flask then hydrochloric acid (275 ml, 12 molar, 1 equivalent) was added and the solution was stirred at 20°C for 10 minutes. 7β-[2′-(R)-2′-phenyl-2′-aminoacetamido]-3-chloro-3-(1-carba-1-dethiacephem)-4-carboxylic acid, mono(dimethylformamide) dihydrate (1465.0 g) was added and the resultant suspension was stirred for 15 minutes. Additional hydrochloric acid (27.5 ml, 0.1 equivalent, 12 molar) was added to the suspension and the suspension was stirred for 20 minutes to effect solution. Carbon black (Darco® G60, 750 ml, approximately 250 g) was added to the solution and the resultant suspension was stirred at 24°C for 30 minutes. The suspension was filtered on a 18.5 cm Büchner funnel containing glass fiber paper and HYFLO® filter and (18.5 cm). The filtered solution was passed over HYFLO® filter aid once more as it was added to the flask and the HYFLO® was rinsed with water (600 ml). The solution was again filtered on a Büchner funnel (11 cm) lined with glass fiber paper. The filtered solution was passed through HYFLO® filter aid and then heated to 47°C over a period of 55 minutes. The pH of the solution was raised slowly to pH of 1.55 by the dropwise addition of triethylamine over a period of 35 minutes. This solution was seeded with 100 mg LY163892 monohydrate. The pH of the seeded solution was raised to 1.8 by the slow addition of triethylamine then the solution was stirred slowly for 1.25 hours. Again, the pH of the solution was raised slowly to 4.8 with stirring while maintaining a temperature of about 50°C. The resultant slurry was stirred for an additional 15 minutes then cooled to 20°C. The slurry was filtered on two 32 cm Büchner funnels containing polypropylene pads over a 30 minute period. The filter in each of the two Büchner funnels was washed with filtered, purified water (500 ml) by first cutting the vacuum to the funnels, allowing the wash to stand for 10 minutes then pulling through the wash by the reapplication of the vacuum. (Two washes on each filter was performed). The filters were covered and vacuum was applied for 12 hours. The dried cakes were placed in a cleaned air oven and dried at 30°C for 48 hours to give 894.5 g, 74.3% yield of crystalline LY163892 monohydrate.

Example 3

LY163892 monohydrate from the corresponding trihydrate

(The trihydrate starting material can be made, for example, by taking the mother liquors and wash (1.5 L) that yielded the monohydrate in Step 2, Procedure B, chilling them to refrigerator temperature and combining the chilled solution with acetonitrile (6 l). The resultant solution was cooled to 0°C over 1 hour. The resultant suspension was filtered over glass fiber paper, and the filter cake was washed with additional acetonitrile (500 ml). The material on the filter cakes was LY163892 trihydrate which may be used without further purification.)

Procedure

Water (1.62 L) and hydrochloric acid (12 molar, 43.0 ml) were combined and stirred at 20 °C. 7β-[2′-(R)-2′-phenyl-2′-aminoacetamido]-3-chloro-3-(1-carba-1-dethiacephem)-4-carboxylic acid trihydrate (200.0 g) was added to the solution and the resultant suspension was stirred for 30 minutes. Additional hydrochloric acid (10 ml) was added and the suspension was stirred for 20 minutes to effect solution. Charcoal (8.0 g) was added to the solution and the resultant suspension was stirred for 30 minutes. The suspension was filtered through a HYFLO® filter aid pad on glass fiber paper (the filter was then washed with 100 ml of water). The filtrate was warmed to 45 °C over a 25 minute period. Triethylamine was slowly added to the filtrate to raise the pH of the solution to 1.8 (the temperature of the filtrate was maintained between 45 and 50 °C during the addition). The solution was seeded with 100 mg of LY163892 monohydrate, resulting in a suspension. The suspension was stirred for 1 hour at 50 °C. The pH of the suspension was raised to 4.8 slowly by the addition of triethylamine. The suspension was stirred for an additional 15 minutes at 50 °C then cooled to 25 °C and stirred for 1 hour. The suspension was filtered on a 24 cm Büchner funnel fitted with a polypropylene pad. The filter cake was washed with water (2X, 250 ml), then the funnel was covered and vacuum was applied to the filter cake overnight. The filter cake was put in a cleaned air dryer at 30 °C for 24 hours to give 119.90 g, 65.9% yield of crystalline LY163892 monohydrate.

Example 4

Hard gelatin capsules are prepared using the following ingredients:

| | Quantity (mg/capsule) |
|---|---|
| LY163892 crystalline monohydrate | 200 |
| Starch flowable powder | 186 |
| Starch flowable powder with silicone 5% | 50 |
| Magnesium stearate | 2.5 |

The above ingredients are mixed and filled into hard gelatin capsules in 438.5 mg quantities.

Example 5

A tablet formula is prepared using the ingredients below:

| | Quantity (mg/tablet) |
|---|---|
| LY163892 crystalline monohydrate | 200 |
| Cellulose, microcrystalline | 200 |
| Silicone dioxide, fumed | 10 |
| Stearic acid | 5 |

The components are blended and compressed to form tablets each weighing 415 mg.

Example 6

Suppositories each containing 200 mg of active ingredient are made as follows:

| | |
|---|---|
| LY163892 crystalline monohydrate | 200 mg |
| Saturated fatty acid glycerides to | 2000 mg |

The active ingredient is passed through a No. 60 mesh U.S. sieve and suspended in the saturated fatty acid glycerides previously melted using the minimum heat necessary. The mixture is then poured into a suppository mold of nominal 2 g capacity and allowed to cool.

Example 7

Ready To-Use Aqueous Suspension 200 mg/5 ml

| | |
|---|---|
| LY163892 crystalline monohydrate | 210 mg |
| Cellulose with sodium carboxy-methylcellulose | 95 mg |
| sucrose | 1.85 g |
| parafens | 3 mg |
| emulsion silicone | 2.5 mg |
| pluronic | 5 mg |
| flavor | 2 mg |
| color | 0.5 mg |
| purified water | q.s. to 5 ml |

The ingredients are sieved. The parafens are dissolved in hot purified water and, after cooling, the other ingredients are added. Sufficient purified water is added to produce the required volume. The suspension can then be passed through a colloid mill or homogenizer to produce a more elegant dispersion.

Example 8

Suspension for Reconstitution

| | |
|---|---|
| LY163892 crystalline monohydrate | 210 mg |
| Sucrose | 3 g |
| Xanthan gum | 5 mg |
| starch modified | 10 mg |
| emulsion silicone | 5 mg |
| sodium lauryl sulfate | 0.5 mg |
| methylcellulose | 2 mg |
| flavor | 0.5 mg |
| dye | |

The ingredients are sieved and mixed in a suitable blender, such as a twin-shell, twin core Nauta®-type, or ribbon blender. To constitute, a sufficient volume of purified water is added to produce the required volume.

## Claims

### Claims for the following Contracting States : AT, BE, CH, DE, FR, GB, IT, LI, LU, NL, SE

**1.** A crystalline monohydrate form of the compound of the Formula (I)

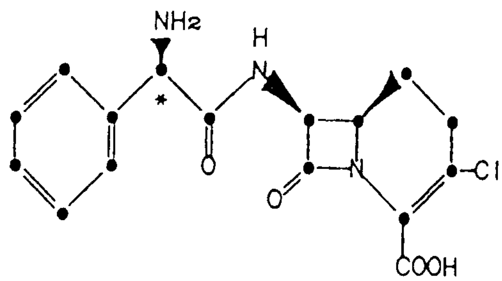

(I)

having the following X-ray powder diffraction pattern:

| d | $I/I_1$ |
|---|---|
| 18.79 | .75 |
| 12.89 | .40 |
| 9.30 | .80 |
| 8.42 | .05 |
| 7.75 | .50 |
| 7.37 | .15 |
| 6.46 | .32 |
| 6.10 | .03 |
| 5.86 | .03 |
| 5.66 | .10 |
| 5.15 | .25 |
| 4.84 | .45 |
| 4.70 | .40 |
| 4.29 | 1.00 |
| 4.06 | .35 |
| 3.89 | .20 |
| 3.77 | .28 |
| 3.69 | .37 |
| 3.53 | .13 |
| 3.49 | .13 |
| 3.39 | .11 |
| 3.24 | .10 |
| 3.13 | .05 |
| 3.05 | .16 |
| 2.95 | .03 |
| 2.90 | .04 |
| 2.85 | .06 |
| 2.77 | .03 |

obtained with nickel-filtered copper radiation (Cu:Ni) of wavelength $\lambda$ = 1.5418 Å, "d" denoting the interplanar spacings and "$I/I_1$" denoting the relative intensities.

**2.** A crystalline monohydrate form of the compound of the Formula (I) as shown in Claim 1, obtainable by adjusting the pH of an aqueous solution of the compound of Formula (I) to about 2 to 6.

**3.** A pharmaceutical formulation comprising as an active ingredient a crystalline monohydrate of the compound of Formula (I) as claimed in either of Claims 1 and 2, associated with one or more pharmaceutically acceptable carriers, vehicles or excipients therefor.

4. A crystalline monohydrate of the compounds of Formula (I) as claimed in either of Claims 1 and 2, for use in treating bacterial infections in warm-blooded animals.

5. A process for preparing the crystalline monohydrate of the compound of Formula (I) as claimed in Claim 1, which comprises adjusting the pH of an aqueous solution containing a compound of Formula (I) to about 2 to 6.

**Claims for the following Contracting States : ES, GR**

1. A process for preparing the crystalline monohydrate form of the compound of the Formula (I)

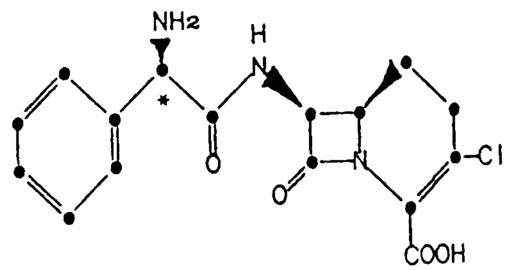

(I)

which comprises adjusting the pH of an aqueous solution containing a compound of Formula (I) to about 2 to 6.

2. A process of Claim 1 wherein the pH is adjusted to about 4 to 5.

3. A process for preparing a pharmaceutical formulation comprising admixing a crystalline monohydrate of the compound of Formula (I) obtainable by a process according to Claim 1, with one or more pharmaceutically acceptable carriers, vehicles or excipients therefor.

**Patentansprüche**
**Patentansprüche für folgende Vertragsstaaten : AT, BE, CH, DE, FR, GB, IT LI, LU, NL, SE**

1. Kristallines Monohydrat der Verbindung der Formel (I)

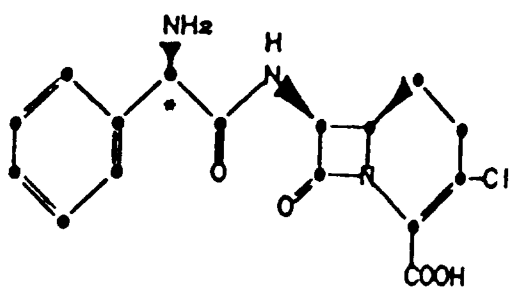

(I)

mit dem folgenden Röntgenbeugungsmuster:

| d | $I/I_1$ |
|---|---|
| 18,79 | 0,75 |
| 12,89 | 0,40 |
| 9,30 | 0,80 |
| 8,42 | 0,05 |
| 7,75 | 0,50 |
| 7,37 | 0,15 |
| 6,46 | 0,32 |
| 6,10 | 0,03 |
| 5,86 | 0,03 |
| 5,66 | 0,10 |
| 5,15 | 0,25 |
| 4,84 | 0,45 |
| 4,70 | 0,40 |
| 4,29 | 1,00 |
| 4,06 | 0,35 |
| 3,89 | 0,20 |
| 3,77 | 0,28 |
| 3,69 | 0,37 |
| 3,53 | 0,13 |
| 3,49 | 0,13 |
| 3,39 | 0,11 |
| 3,24 | 0,10 |
| 3,13 | 0,05 |
| 3,05 | 0,16 |
| 2,95 | 0,03 |
| 2,90 | 0,04 |
| 2,85 | 0,06 |
| 2,77 | 0,03 |

erhalten mit einer durch Nickel gefilterten Kupferstrahlung (Cu:Ni) der Wellenlänge $\lambda$ = 1,5418 Å, wobei "d" die Ebenenabstände bezeichnet und "$I/I_1$" die relativen Intensitäten bezeichnet.

**2.** Kristallines Monohydrat der Verbindung der Formel (I), wie in Anspruch 1 gezeigt, erhältlich, indem der pH einer wäßrigen Lösung der Verbindung der Formel (I) auf etwa 2 bis 6 eingestellt wird.

**3.** Pharmazeutisches Präparat umfassend als aktiven Inhaltsstoff ein kristallines Monohydrat der Verbindung der Formel (I), wie in einem der Ansprüche 1 oder 2 beansprucht, zusammen mit einem oder mehreren pharmazeutisch annehmbaren Trägern oder Hilfsstoffen dafür.

**4.** Kristallines Monohydrat der Verbindungen der Formel (I) nach einem der Ansprüche 1 oder 2 zur Verwendung zur Behandlung von bakteriellen Infektionen bei Warmblütern.

5. Verfahren zur Herstellung des kristallinen Monohydrats der Verbindung der Formel (I), wie in Anspruch 1 beansprucht, umfassend, daß man den pH einer wäßrigen Lösung, die eine Verbindung der Formel (I) enthält, auf etwa 2 bis 6 einstellt.

**Patentansprüche für folgende Vertragsstaaten : ES, GR**

1. Verfahren zur Herstellung des kristallinen Monohydrats der Verbindung der Formel (I)

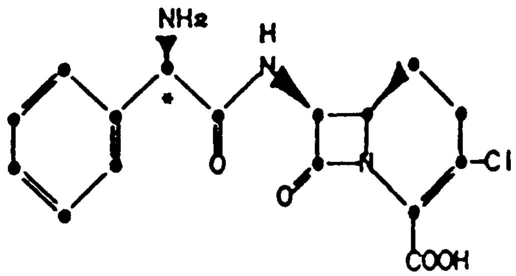

(I)

umfassend, daß man den pH einer wäßrigen Lösung, die eine Verbindung der Formel (I) enthält, auf etwa 2 bis 6 einstellt.

2. Verfahren nach Anspruch 1, worin der pH auf etwa 4 bis 5 eingestellt wird.

3. Verfahren zur Herstellung eines pharmazeutischen Präparates umfassend, daß man ein kristallines Monohydrat der Verbindung der Formel (I), die erhältlich ist mit einem Verfahren gemäß Anspruch 1, mit einem oder mehreren pharmazeutisch annehmbaren Trägern oder Hilfsstoffen dafür vermischt.

**Revendications**

**Revendications pour les Etats contractants suivants : AT, BE, CH, DE, FR, GB, IT, LI, LU, NL, SE**

1. Forme monohydratée cristalline du composé de formule (I)

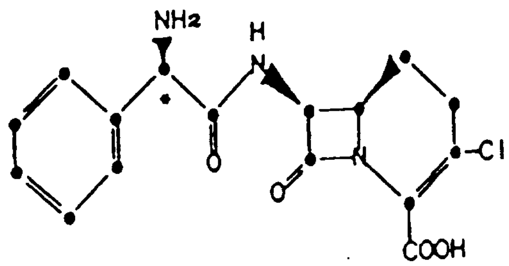

(I)

possédant le radiogramme de poudre ci-après :

| d | $I/I_1$ |
|---|---|
| 18,79 | 0,75 |
| 12,89 | 0,40 |
| 9,30 | 0,80 |
| 8,42 | 0,05 |
| 7,75 | 0,50 |
| 7,37 | 0,15 |
| 6,46 | 0,32 |
| 6,10 | 0,03 |
| 5,86 | 0,03 |
| 5,66 | 0,10 |
| 5,15 | 0,25 |
| 4,84 | 0,45 |
| 4,70 | 0,40 |
| 4,29 | 1,00 |
| 4,06 | 0,35 |
| 3,89 | 0,20 |
| 3,77 | 0,28 |
| 3,69 | 0,37 |
| 3,53 | 0,13 |
| 3,49 | 0,13 |
| 3,39 | 0,11 |
| 3,24 | 0,10 |
| 3,13 | 0,05 |
| 3,05 | 0,16 |
| 2,95 | 0,03 |
| 2,90 | 0,04 |
| 2,85 | 0,06 |
| 2,77 | 0,03 |

obtenu par rayonnement de cuivre filtré au nickel (Cu:Ni) de longueur d'onde lambda = 1,5418 Å, "d" désignant les écartements des plans et "$I/I_1$ " désignant les intensités relatives.

**2.** Forme monohydratée cristalline du composé de formule (I), tel que représenté dans la revendication 1, que l'on obtient en réglant le pH d'une solution aqueuse du composé de formule (I) à une valeur d'environ 2 à 6.

**3.** Formulation pharmaceutique comprenant, comme ingrédient actif, un monohydrate cristallin du composé de formule (I) selon l'une quelconque des revendications 1 et 2, en association avec un ou plusieurs supports, véhicules ou excipients pharmaceutiquement acceptables pour l'ingrédient actif.

**4.** Monohydrate cristallin du composé de formule (I) selon l'une quelconque des revendications 1 et 2, pour être utilisé dans le traitement d'infections bactériennes chez les animaux à sang chaud.

**5.** Procédé pour préparer le monohydrate cristallin du composé de formule (I) selon la revendication 1, qui comprend le réglage du pH d'une solution aqueuse contenant un composé de formule (I) à une valeur d'environ 2 à 6.

**Revendications pour les Etats contractants suivants : ES, GR**

1. Procédé pour préparer la forme monohydratée cristalline du composé de formule (I)

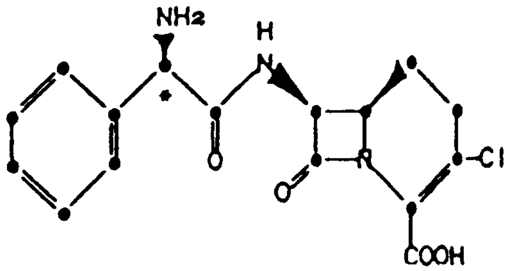

(I)

qui comprend le réglage du pH d'une solution aqueuse contenant un composé de formule (I) à une valeur d'environ 2 à 6.

2. Procédé selon la revendication 1, dans lequel le pH est réglé à une valeur d'environ 4 à 5.

3. Procédé pour préparer une formulation pharmaceutique, comprenant le mélange d'un monohydrate cristallin du composé de formule (I), que l'on obtient par un procédé selon la revendication 1, avec un ou plusieurs supports, véhicules ou excipients pharmaceutiquement acceptables pour le composé.

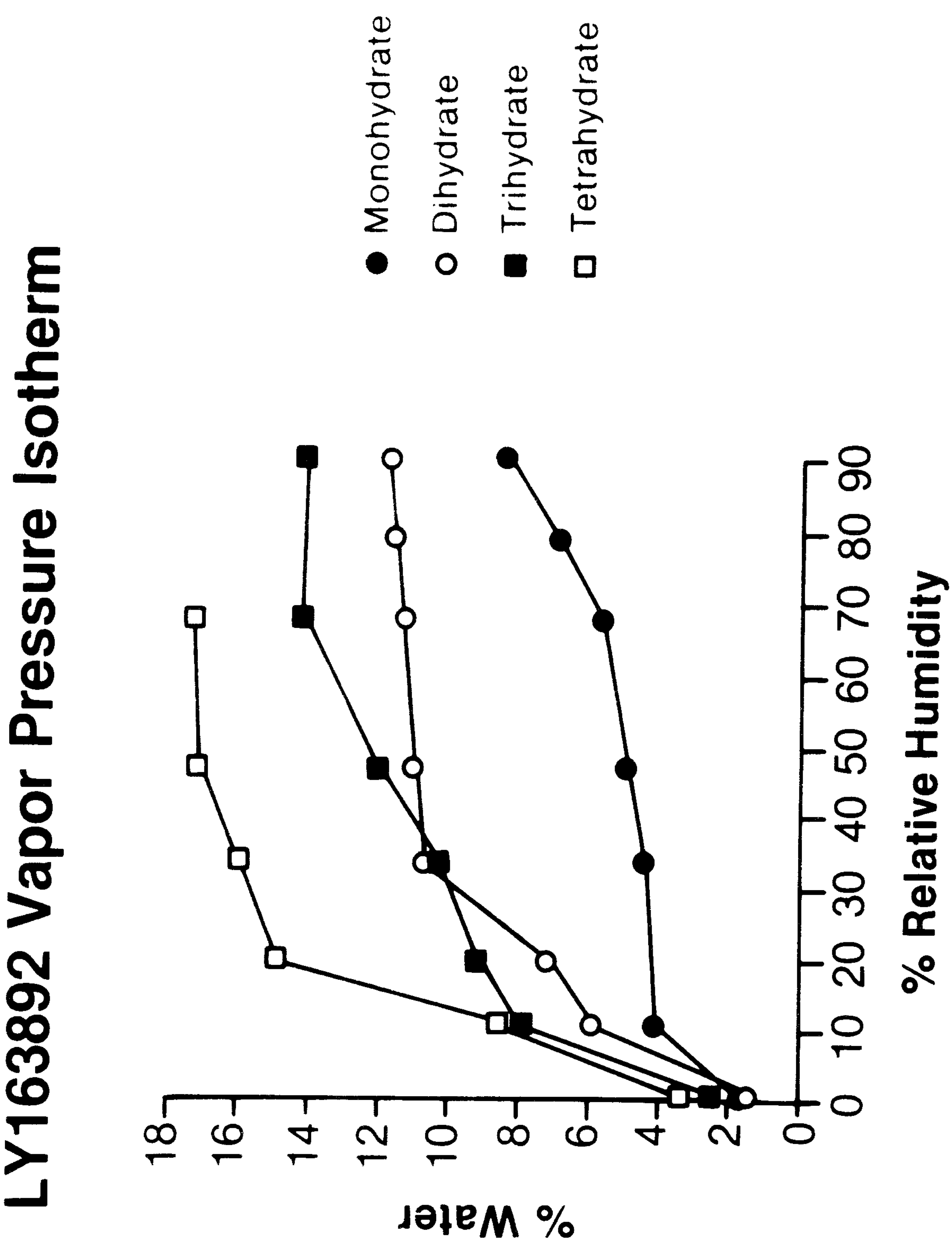
LY163892 Vapor Pressure Isotherm
% Water
0
2
4
6
8
10
12
14
16
18
% Relative Humidity
0
10
20
30
40
50
60
70
80
90
Monohydrate
Dihydrate
Trihydrate
Tetrahydrate